# EUROPÄISCHE PATENTSCHRIFT

(11) **EP 1 159 621 B1**
(45) Veröffentlichungstag und Bekanntmachung des Hinweises auf die Patenterteilung: **27.10.2004**
(21) Anmeldenummer: 00912545.1
(22) Anmeldetag: 03.03.2000
(51) Int. Cl.: G01N 33/68, G01N 33/543, G01N 33/569

(54) **VERFAHREN ZUR BESTIMMUNG DER AVIDITÄT VON ANTIKÖRPERN**
METHOD FOR DETERMINING THE AVIDITY OF ANTIBODIES
PROCEDE PERMETTANT DE DETERMINER L'AVIDITE D'ANTICORPS

(30) Priorität: 08.03.1999 DE 19910045
(43) Veröffentlichungstag der Anmeldung: 05.12.2001
(73) Patentinhaber: Mikrogen Molekularbiologische Entwicklungs-GmbH, 82152 Martinsried (DE)
(72) Erfinder: BAUER, Georg, D-79104 Freiburg (DE)
(74) Vertreter: Keller, Günter, Dr.
(86) Internationale Anmeldenummer: PCT/EP2000/001883
(87) Internationale Veröffentlichungsnummer: WO 2000/054055

(56) Entgegenhaltungen:
- EP-A- 0 875 761
- WO-A-90/02202
- WO-A-97/09619
- US-A- 5 679 537
- SODERLUND M. ET AL JOURNAL OF INFECTIOUS DISEASES Bd. 171, Nr. 3, 1995, Seiten 710 - 713

## Beschreibung

Die vorliegende Erfindung betrifft ein Verfahren, mit dem in einem Versuchsansatz die Aviditäten der Antikörper gegen mehrere Antigene bestimmt werden können.

Eine wichtige Aufgabe der serologischen Diagnostik besteht darin, festzustellen, ob im konkreten Fall eine akute Infektion vorliegt oder nicht. In der Regel ist eine Primärinfektion durch eine transient auftretende IgM-Antwort gekennzeichnet, während die IgG-Antwort lange andauert. Auf diesen Beobachtungen basiert die klassische Infektionsserologie, nach der Erreger-spezifische IgG und IgM bestimmt werden. So ergibt sich aufgrund eines IgM-Befundes ein Hinweis auf eine akute Infektion, während ein IgG-Befund ohne paralleles IgM auf eine länger zurückliegende Infektion hindeutet. Aufgrund der Variabilität der Immunantwort und durch das Auftreten aberranter serologischer Verläufe kann jedoch diese klassische Vorgehensweise in nicht wenigen Fällen zu Fehlschlüssen führen.

So kann aufgrund einer persistierenden oder reaktivierten IgM-Antwort eine Primärinfektion vorgetäuscht werden. Ebenso kann zum Beispiel eine Epstein-Barr-Virus (EBV)-Infektion zu polyklonaler B-Zellstimulierung führen, in deren Folge nach dem Zufallsprinzip IgM-Moleküle synthetisiert werden, ohne daß der Organismus Kontakt mit dem entsprechenden Erreger hatte. Aufgrund der hohen Variabilität der IgM-Antwort sind jedoch auch falschnegative Ergebnisse möglich.

Bei der Ausbildung der IgG-Antwort kommt es zu einer sukzessiven Zunahme der Affinität des gebildeten IgGs für das entsprechende Antigen. Diese Affinitätsreifung ist ein äußerst regelmäßig auftretender immunologischer Prozeß. Daher liegt bei einer akuten Infektion stets ein niedrigaffines IgG, bei einer abgelaufenen Infektion hochaffines IgG vor.

Aus diesem Grund ist zur Lösung der oben genannten Probleme in den letzten Jahren ein weiterer, sehr aussagekräftiger Marker in die Diagnostik eingeführt worden: die Avidität des IgG. Die Avidität ist ein Maß für die Stärke der Bindung zwischen einem Antikörper und "seinem" Antigen. Anhand der EBV-Diagnostik ist die hohe Aussagekraft dieses Verfahrens, die die der klassischen Serologie weit übersteigt, exemplarisch belegt worden (Andersson et al. (1994) J. Med. Virol. 43, 238-244; Vetter et al. (1994) Clin. Diag. Virol. 2, 29-39; Bauer (1994) Therapeutische Umschau 51, 558-562; Bauer (1995) Clin. Lab. 41, 623-634; Wolters et al. (1997) Clin. Lab. 43, 125-135). Allerdings verläuft die Reifung der Avidität von IgGs verschiedener Antigene nicht unbedingt synchron. So zeigte sich zum Beispiel, daß die Aviditätsreifung des IgG gegen "virus capsid antigens" (VCA) schon abgeschlossen ist, wenn die Avidität des IgG gegen die Komponenten des "early antigen" (EA)-Komplexes noch am Ansteigen ist. Aus dieser Tatsache ergibt sich das Erfordernis, mehrere Antigene eines Erregers zu testen, um eine zuverlässige Aussage über den Stand einer Infektion zu erhalten.

Bekannte Verfahren zur Bestimmung der Avidität sind der ELISA oder der Immunfluoreszenztest. Beim Immunfluoreszenztest kann jedoch immer nur ein Antigen untersucht werden, beim ELISA müßten alle Antigene in Einzel-Vergleichstests gemessen werden. Bei einem Gesamtansatz mit mehreren Antigenen im Immunfluoreszenztest oder ELISA würde man immer einen Durchschnittswert der Aviditätsreduktion nach Harnstoff-Behandlung erhalten. EP 0 875 761 offenbart ein Verfahren zur Bestimmung der Avidität eines Antikörpers, bei dem Harnstoff-Wasserstoffperoxid eingesetzt wird. Söderlund et al. [J. of Inf. Dis. (1995), S. 710-713] beschreiben die Verwendung von rekombinanten Parvovirus-Antigenen zur Aviditätsmessung in Einzelansätzen.

Eine Aufgabe der vorliegenden Erfindung besteht darin, ein Verfahren bereitzustellen, mit dem in einem einzigen Versuchsansatz die Aviditäten der Antikörper gegen mehrere definierte Antigene bestimmt werden können.

Die Aufgabe wurde durch das erfindungsgemäße Verfahren gelöst. Dabei werden ein oder mehrere verschiedene Antigene auf einer festen Phase immobilisiert, wobei die verschiedenen Antigene räumlich voneinander getrennt sind. Es werden insgesamt mindestens zwei feste Phasen hergestellt, auf denen die Proteine in gleicher Weise und in gleicher Menge verteilt sind. Diese festen Phasen werden nun mit einer Antikörperhaltigen Lösung inkubiert. Anschließend wird die erste feste Phase in einer Lösung inkubiert, die die meisten Antikörper-Antigen-Wechselwirkungen nicht beeinträchtigt (nicht-stringente Lösung). Die anderen festen Phasen jedoch werden in einer Lösung inkubiert, die ein Mittel enthält, das niedrigavide Antikörper von ihren Antigenen ablöst (stringente Lösung). Nach Waschen werden schließlich die noch an die festen Phasen gebundenen Antikörper nachgewiesen. Durch Vergleich der festen Phasen, die in nicht-stringenter Lösung inkubiert wurden, mit den festen Phasen, die in stringenter Lösung inkubiert wurden, kann für jedes Antigen die Avidität bestimmt werden. Auf jeder festen Phase sind bevorzugt wenigstens zwei verschiedene, definierte Antigene immobilisiert, bevorzugter wenigstens drei verschiedene, definierte Antigene, am bevorzugtesten wenigstens vier verschiedene, definierte Antigene. Es ist aber auch denkbar, daß fünf, sechs oder mehr verschiedene Antigene auf jeder festen Phase immobilisiert sind. Der Vorteil des beschriebenen Verfahrens liegt darin, daß in einem Experiment die Aviditäten von zahlreichen Antikörpern bestimmt werden können. Dadurch lassen sich "Aviditätsprofile" gewinnen, die charakteristisch für die Dauer der Infektion sind. Das ermöglicht eine genauere Aussage als es bisher möglich war. Wenn mehr als zwei feste Phasen eingesetzt werden, können die weiteren festen Phasen in Lösungen steigender Stringenz inkubiert werden, so daß ein differenziertes Bild über die Avidität zahlreicher Antikörper erhalten werden kann. Das stellt einen weiteren Vorteil des erfindungsgemäßen Verfahrens dar.

Die Zusammensetzungen der stringenten und der nicht-stringenten Lösung sind nicht besonders eingeschränkt. Sie sind aber derart, daß an die feste Phase, die mit der nicht-stringenten Lösung inkubiert wurde, nach der Inkubation mehr Antikörper gebunden sind als an die feste Phase, die mit der stringenten Lösung inkubiert wurde. Die nicht-stingente Lösung sowie die Waschlösungen können eine Puffersubstanz wie Phosphat oder Tris-HCl oder Hepes enthalten. Weiterhin enthalten diese Lösungen bevorzugt bis zu 500mM, bevorzugter bis zu 250mM, am bevorzugtesten etwa 150 mM Salz. Als Salze kommen anorganische Salze wie NaCl, KCl, MgCl₂ oder ähnliche Salze in Betracht. Eine bevorzugte nicht-stringente Lösung ist PBS oder TBS, gegebenenfalls mit weiteren Zusätzen. Die stringente Lösung kann die Bestandteile der nicht-stringenten Lösung umfassen, wobei wenigstens ein weiteres Mittel enthalten ist, das die Bindung niedrig-avider Antikörper an die Antigene schwächt. In einer bevorzugten Ausführungsform des Verfahrens enthält die stringente Lösung eine denaturierende Substanz in einer Konzentration, die geeignet ist, die Bindung von Antikörpern an Antigene wesentlich zu schwächen.

In einer bevorzugten Ausführungsform des erfindungsgemäßen Verfahrens enthält die stringente Lösung Harnstoff, vorzugsweise in einer Konzentration von 4-9 M, bevorzugt in einer Konzentration von 4,8 bis 7,5 M und am bevorzugtesten in einer Konzentration von etwa 5,5 M. Neben Harnstoff kann jedes in ähnlicher Weise denaturierende Agens verwendet werden. Beispiele dafür sind Diethylamin, Guanidin oder Thiocyanat.

Die festen Phasen können Kunststoffflächen wie Polystyrolkugeln oder Mikrotiterplatten sein, vorzugsweise sind sie jedoch Membranen. Die festen Phasen können auch in Form von "Biochips" ausgebildet sein oder bei Plattformen für Multiparameter-Bestimmungen eingesetzt werden.

In bevorzugter Weise bestehen die Membranen aus Nitrocellulose, Nylon oder Polyvinylidenfluorid (PVDF).

Die Antigene können direkt auf die Membranen aufgetragen werden. Bevorzugt werden die Membranen jedoch durch Gelelektrophorese der Antigene und anschließenden elektrophoretischen Transfer auf die Membranen hergestellt. Dadurch entstehen Membranen, auf denen die Proteine entsprechend ihrer Größe aufgetrennt sind. Vorzugsweise wurden die Antigene vor der Immobilisierung auf der festen Phase gereinigt. Dabei kann es sich um "native" Proteine oder Proteinfragmente handeln, die aus dem Erreger isoliert wurden, es kann sich aber auch um rekombinant hergestellte Antigene handeln. Dem Fachmann ist klar, daß ein Antigen nicht unbedingt die vollständige Aminosäuresequenz des "nativen" Proteins umfassen muß.

Die bevorzugte Detektionsmethode beruht auf der Bindung von sekundären enzymgekoppelten Antikörpern an die primären membrangebundenen Antikörper und nachfolgender Visualisierung durch enzymatische umsetzung eines Substrats.

Die beschriebenen Verfahren werden bevorzugt in der Diagnostik eingesetzt. In einem Testkit könnten beispielsweise Membranstreifen enthalten sein, die mehrere Antigene des EBV enthalten. Außerdem kann eine denaturierende Substanz enthalten sein, vorzugsweise Harnstoff, die als Mittel in den stringenten Lösungen eingesetzt wird. Darüber hinaus können in einem solchen Testkit Reagenzien zur Durchführung einer "Western-Blot"-Detektion von Proteinen enthalten sein.

Ein bevorzugtes Testkit kann mehrere Western-Blot-Streifen oder andere Träger enthalten, auf denen Antigene eines Erregers immobilisiert sind. Die Antigene können beispielsweise vom EBV, von humanem Cytomegalovirus (HCMV), von Hepatitis-Erregern, wie Hepatitis A, B, C oder G, oder von humanem Immundefizienz-Virus (HIV) stammen. Auch die Antigene von anderen Erregern, wie Bakterien, Pilzen oder Protozoen, können eingesetzt werden. Weiterhin ist üblicherweise ein Waschpuffer enthalten, der Protein enthält und somit auch zum Blockieren unspezifischer Bindestellen auf den Membranen eingesetzt werden kann. Er kann auch zur Probenverdünnung eingesetzt werden. Das Testkit enthält üblicherweise auch Seren, die als Positiv- bzw. Negativkontrolle dienen. Die stringente Lösung kann in Form einer vorbereiteten Harnstofflösung enthalten sein. Schließlich kann das Kit sekundäre Antikörper enthalten, die gegen humane IgG gerichtet sind und mit einem Enzym, beispielsweise Meerrettich-Peroxidase, konjugiert sind.

Weiterhin können in einem Testkit Membranstreifen oder Träger enthalten sein, die Antigene verschiedener Erreger tragen. Dadurch kann bei der Diagnose eine größere Bandbreite abgedeckt werden. Bei derartigen Testkits können beispielsweise Antigene verschiedener Krankheitserreger aufgetragen werden, um zum einen in einem Ansatz den serologischen Status zu bestimmen und gleichzeitig mittels Aviditätstest zu unterscheiden, ob es sich hierbei um akute, frische Infektionen, Reaktivierung oder unspezifische Stimulierung handelt.

Dies ist wichtig bei bestimmten viralen Erkrankungen (z.B. EBV, CMV), die eine unspezifische Stimulierung des Immunsystems bewirken und damit oft das serologische Bild einer Frischinfektion für andere Parameter anzeigen - mit dem Unterschied, daß bei diesen dann nur hochavide Antikörper vorhanden sind.

Insbesonders bei Patienten mit Störung des Immunsystems oder immunsuppressiv behandelten Patienten ist dieser Ansatz ebenfalls sinnvoll, da es hier vielfach zu Reaktivierung persistierender Viren (EBV, CMV, evtl. Parvovirus B19 u.a.) mit dem serologischen Bild einer Frischinfektion kommen kann. Diese Reaktivierungen können mit einem solchen Aviditätstest in einem Ansatz von eventuell vorhandenen zusätzlichen Infektionen unterschieden werden.

Die folgenden Beispiele sollen das erfindungsgemäße Verfahren näher erläutern:

### Beispiel 1

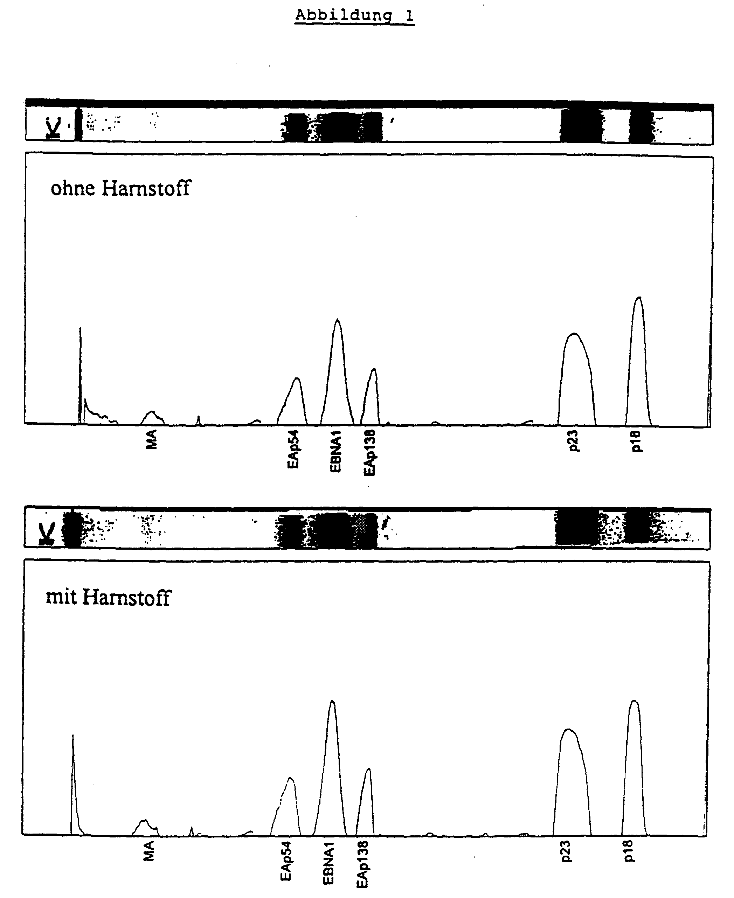

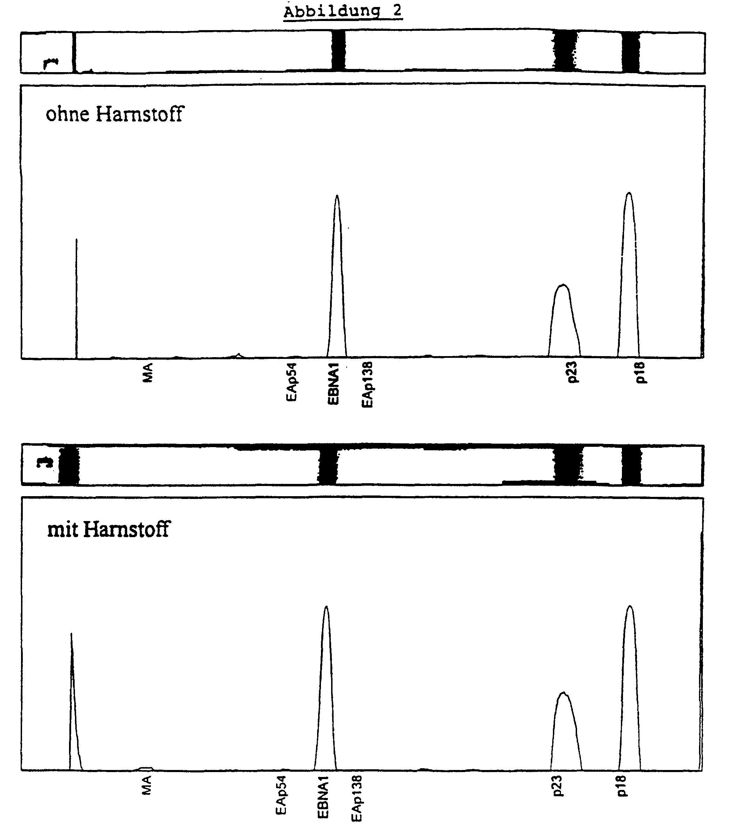

Die rekombinanten EBV-Antigene MA, p18, p23, p54, p138 und EBNA-1 (p72) werden durch Gelelektrophorese aufgetrennt und anschließend elektrophoretisch auf eine Nitrozellulosemembran transferiert. Nach Absättigung unspezifischer Bindungsstellen auf der Membran wird diese in Streifen geschnitten. Zwei dieser Membranstreifen werden in unterschiedlichen Inkubationsvertiefungen (Ansatz 1 und 2) mit dem gleichen Serum (1:100 verdünnt in Salz/Protein-haltigem Waschpuffer) für drei Stunden unter leichtem Schütteln bei Raumtemperatur inkubiert. Die verdünnten Seren werden anschließend abgesaugt und Ansatz 1 mit Waschpuffer versetzt, während zu Ansatz 2 5,5M Harnstoff (in PBS) gegeben wird. Die Streifen werden für 3 min inkubiert und anschließend jeweils viermal für fünf Minuten mit Waschpuffer unter Schütteln gewaschen. Anschließend werden beide Membranstreifen für 1 Stunde mit Anti-Human-IgG-Kaninchenantikörper (1:1000 verdünnt; Peroxidase-konjugiert) inkubiert. Anschließend wird wiederum viermal gewaschen, und die gebundenen Antikörper werden durch enzymatische Färbung mittels TMB oder DAB nachgewiesen. Die Reaktion wird gestoppt durch Waschen der Streifen mit Wasser. Es ist wichtig, daß die Färbereaktion für beide Streifen unter gleichen Bedingungen durchgeführt wird. Nach dem Trocknen der Membranen werden die Streifen densitometrisch analysiert. Die dabei ermittelten Intensitäten der Banden von Harnstoffbehandelten Streifen und Kontrollstreifen werden verglichen. Aus den unterschiedlichen Intensitäten für die Einzelantigene läßt sich ein Aviditätsindex berechnen (Quotient Intensität nach Harnstoff-Behandlung/Intensität ohne Harnstoff-Behandlung). Dieser Index erlaubt eine zusätzliche Aussage über die Frage, ob eine Frischinfektion oder eine zurückliegende Infektion vorliegt. Abbildung 1 und 2 zeigen die Ergebnisse von Versuchen, in denen die Seren von zwei Personen mit abgelaufener EBV-Infektion verwendet wurden. Alle erkannten Marker haben hochavide Antikörper, denn durch die Harnstoff-Behandlung ergibt sich keine Abschwächung der Banden. Dadurch ist die abgelaufene EBV-Infektion charakterisiert.

### Beispiel 2

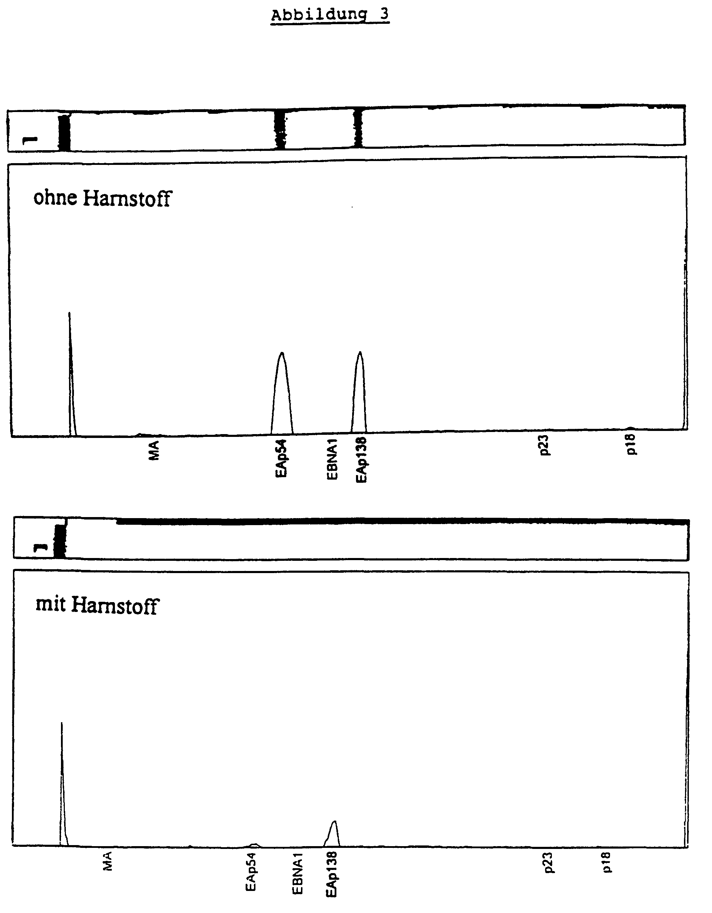

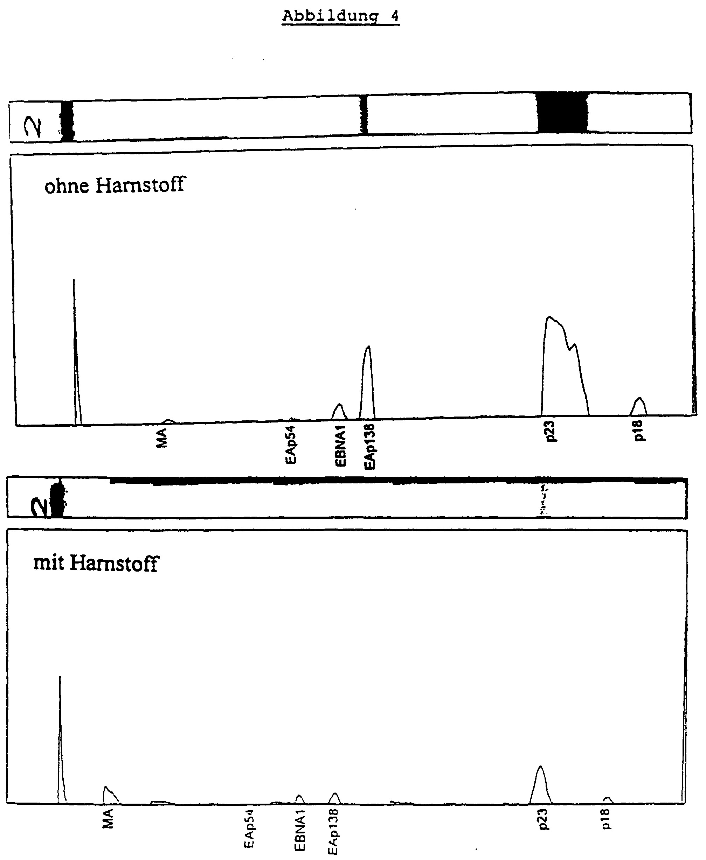

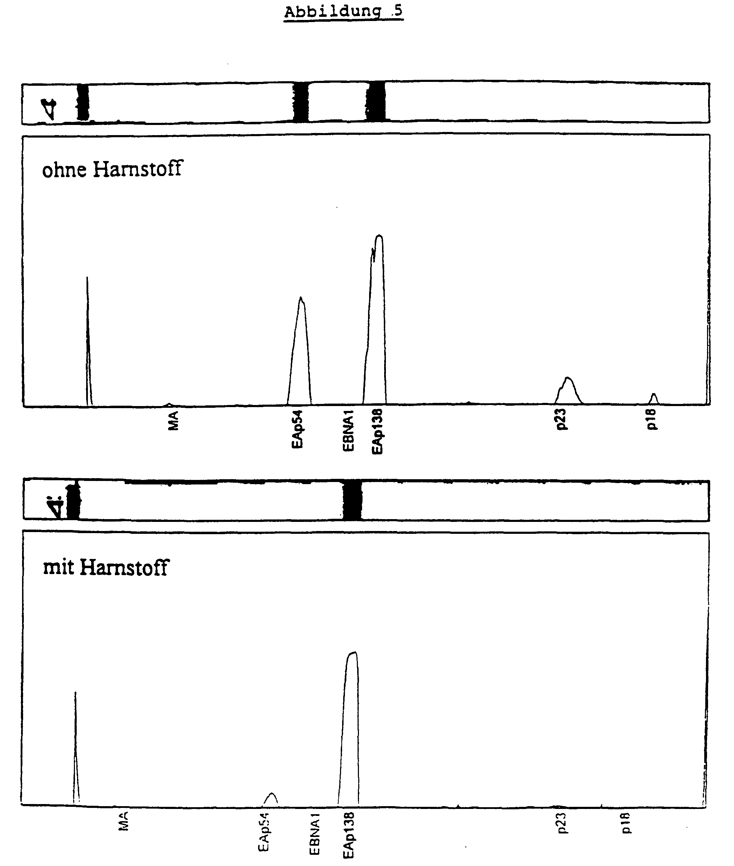

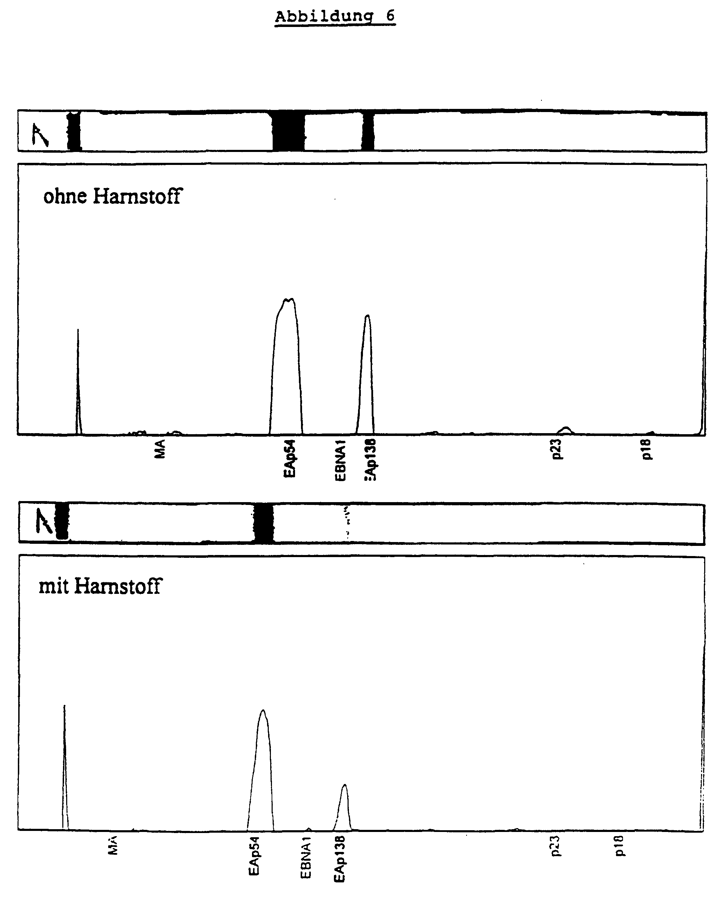

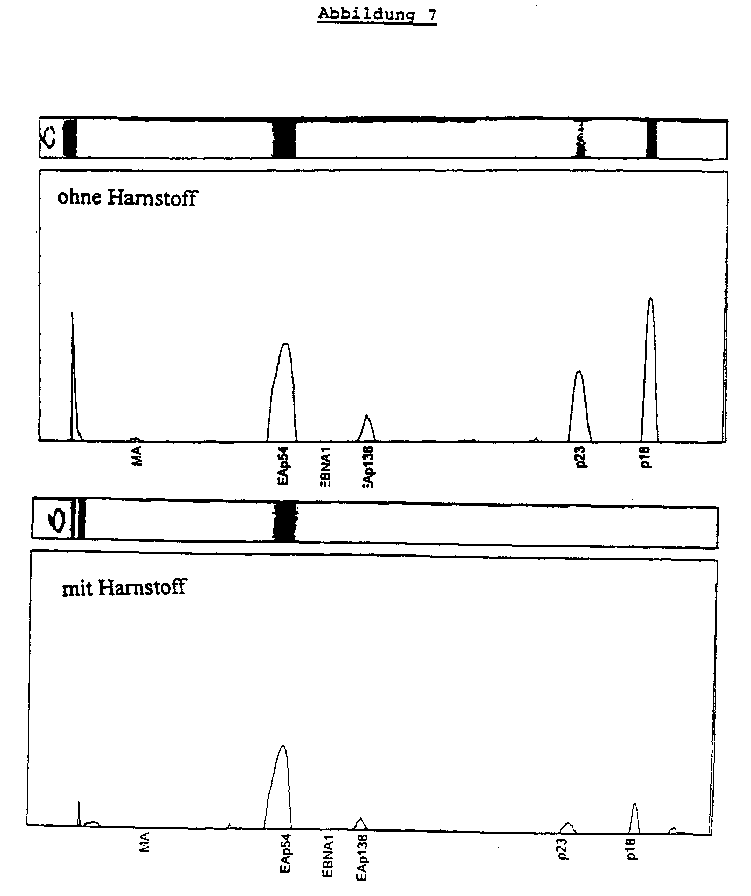

Es wurden Membranstreifen wie in Beispiel 1 hergestellt, wobei Seren von Patienten mit frischer EBV-Infektion getestet wurden. Die Versuche wurden durchgeführt, wie in Beispiel 1 beschrieben. Abbildung 3 zeigt das Ergebnis eines Serums mit niedrigaviden Antikörpern gegen p54 und p138. Dies zeigt eine frische Infektion. Abbildung 4 zeigt niedrigavide Antikörper gegen p23 und p138. Dies zeigt ebenfalls frische Infektion. In Abbildung 5 ist erkennbar, daß Antikörper gegen p138 schon gereift sind (hochavide), aber die niedrige Avidität der Antikörper gegen p54 und p23 läßt die frische EBV-Infektion erkennen. Hier wird der Wert dieses Verfahrens besonders erkennbar: Bei alleiniger Testung der Avidität von p138 wäre ein falscher Ausschluß einer frischen Infektion erfolgt. In Abbildung 6 läßt die Reduktion der p54-Bande und der p138-Bande durch Harnstoff auf eine kürzliche Infektion schließen. Die Aviditätsreifung hat bereits begonnen, ist aber noch nicht abgeschlossen. Schließlich ist in Abbildung 7 IgG gegen p54 bereits weitgehend gereift, während die niedrige Avidität der Antikörper gegen p23 und p18 die frische EBV-Infektion erkennen läßt.

## Patentansprüche

1. Verfahren zur Bestimmung der Avidität von Antikörpern, das folgende Schritte umfaßt:
a) Bereitstellung von mindestens zwei festen Phasen, wobei auf jeder festen Phase in gleicher Weise und in gleicher Menge wenigstens zwei verschiedene Antigene immobilisiert sind, und auf jeder festen Phase die verschiedenen Antigene räumlich voneinander getrennt sind,
b) Inkubation der festen Phasen aus a) mit einer Antikörper enthaltenden Lösung,
c) Inkubation einer der festen Phasen in einer Lösung, die nicht die Bindung von Antikörpern mit niedriger Avidität an die Antigene wesentlich reduziert,
d) Inkubation mindestens einer weiteren festen Phase in einer Lösung, wobei diese Lösung ein Mittel aufweist, das die Bindung von Antikörpern mit niedriger Avidität an die Antigene reduziert, und
e) Detektion der an die festen Phasen gebundenen Antikörper.

2. Verfahren nach Anspruch 1, **dadurch gekennzeichnet, daß** das in Schritt d) eingesetzte Mittel wenigstens eine Proteine denaturierende Substanz enthält.

3. Verfahren nach Anspruch 2, **dadurch gekennzeichnet, daß** das Mittel Harnstoff enthält.

4. Verfahren nach Anspruch 3, **dadurch gekennzeichnet, daß** Harnstoff in einer Konzentration von 4 bis 9 M verwendet wird.

5. Verfahren nach einem der Ansprüche 1 bis 4, **dadurch gekennzeichnet, daß** die festen Phasen Membranen sind.

6. Verfahren nach Anspruch 5, **dadurch gekennzeichnet, daß** die Membranen aus einem Material bestehen, ausgewählt aus einer Gruppe enthaltend Nitrocellulose, Nylon und Polyvinylidenfluorid.

7. Verfahren nach Anspruch 5 oder 6, **dadurch gekennzeichnet, daß** die Membranen durch Gelelektrophorese und anschließenden elektrophoretischen Transfer der Antigene auf die Membranen bereitgestellt werden.

8. Verfahren nach einem der Ansprüche 1 bis 7, **dadurch gekennzeichnet, daß** die Detektion der Antikörper in Schritt e) durch Inkubation mit enzymgekoppelten sekundären Antikörpern und anschließende enzymatische Nachweisreaktion erfolgt.

9. Verfahren nach einem der vorhergehenden Ansprüche, **dadurch gekennzeichnet, daß** die Avidität von Antikörpern gegen Epstein-Barr-Virus bestimmt wird.

10. Verfahren nach einem der vorhergehenden Ansprüche, **dadurch gekennzeichnet, daß** auf den festen Phasen gereinigte Proteine immobilisiert sind.

11. Verfahren nach einem der vorhergehenden Ansprüche, **dadurch gekennzeichnet, daß** die auf den festen Phasen immobilisierten Antigene rekombinant hergestellt werden.

12. Verfahren nach einem der vorhergehenden Ansprüche, **dadurch gekennzeichnet, daß** an der festen Phase, die nach c) inkubiert wurde, nach der Inkubation mehr Antikörper gebunden sind als an der festen Phase, die nach d) inkubiert wurde.

13. Verfahren nach einem der vorhergehenden Ansprüche, **dadurch gekennzeichnet, daß** die Lösung zur Inkubation nach c) wenigstens eine Puffersubstanz und bis zu 500 mM Salz, bevorzugt bis zu 250 mM Salz, enthält.

14. Testkit für die Durchführung eines Verfahrens nach einem der Ansprüche 1 bis 13, **dadurch gekennzeichnet, daß** es mehrere Western-Blot-Streifen oder andere Träger enthält, auf denen Antigene eines Erregers immobilisiert sind, wobei auf jedem Träger in gleicher Weise und in gleicher Menge wenigstens zwei verschiedene Antigene immobilisiert sind, und auf jedem Träger die verschiedenen Antigene räumlich voneinander getrennt sind.

15. Testkit nach Anspruch 14, **dadurch gekennzeichnet, daß** es ein denaturierendes Mittel enthält.

16. Testkit nach Anspruch 15, **dadurch gekennzeichnet, daß** es als denaturierendes Mittel Harnstoff enthält.

17. Verwendung rekombinant hergestellter Antigene zur Bestimmung der Aviditäten von Antikörpern gegen Antigene, wobei
a) mindestens zwei feste Phasen bereitgestellt werden, auf jeder festen Phase in gleicher Weise und in gleicher Menge wenigstens zwei verschiedene Antigene immobilisiert sind, und auf jeder festen Phase die verschiedenen Antigene räumlich voneinander getrennt sind,
b) die festen Phasen aus a) mit einer Antikörper enthaltenden Lösung inkubiert werden,
c) eine der festen Phasen in einer Lösung, die nicht die Bindung von Antikörpern mit niederiger Avidität an die Antigene wesentlich reduziert, inkubiert wird,
d) mindestens eine weitere feste Phase in einer Lösung inkubiert wird, wobei diese Lösung ein Mittel aufweist, das die Bindung von Antikörpern mit niedriger Avidität an die Antigene reduziert, und
e) die an die festen Phasen gebundenen Antikörper detektiert werden.

## Claims

1. A method for determining the avidity of antibodies, which comprises the following steps:
a) provision of at least two solid phases, wherein at least two different antigens are immobilized in the same way and in the same quantity on each solid phase, the different antigens on each solid phase being spatially separate from one another,
b) incubation of the solid phases from a) with an antibody-containing solution,
c) incubation of one of the solid phases in a solution which negligibly reduces the binding of antibodies with low avidity to the antigens,
d) incubation of at least one other solid phase in a solution, where this solution comprises an agent which reduces the binding of antibodies with low avidity to the antigens, and
e) detection of the antibodies bound to the solid phases.

2. The method as claimed in claim 1, **characterized in that** the agent employed in step d) comprises at least one protein-denaturing substance.

3. The method as claimed in claim 2, **characterized in that** the agent comprises urea.

4. The method as claimed in claim 3, **characterized in that** the urea is used in a concentration of from 4 to 9 M.

5. The method as claimed in any of claims 1 to 4, **characterized in that** the solid phases are membranes.

6. The method as claimed in claim 5, **characterized in that** the membranes consist of a material selected from a group comprising nitrocellulose, nylon and polyvinylidene fluoride.

7. The method as claimed in claim 5 or 6, **characterized in that** the membranes are provided by gel electrophoresis and subsequent electrophoretic transfer of the antigens onto the membranes.

8. The method as claimed in any of claims 1 to 7, **characterized in that** the detection of the antibodies in step e) takes place by incubation with enzyme-coupled secondary antibodies and subsequent enzymatic detection reaction.

9. The method as claimed in any of the preceding claims, **characterized in that** the avidity of antibodies against Epstein-Barr virus is determined.

10. The method as claimed in any of the preceding claims, **characterized in that** purified proteins are immobilized on the solid phases.

11. The method as claimed in any of the preceding claims, **characterized in that** the antigens immobilized on the solid phases have been produced recombinantly.

12. The method as claimed in any of the preceding claims, **characterized in that** more antibodies are bound after the incubation to the solid phase which was incubated as in c) than to the solid phase which was incubated as in d).

13. The method as claimed in any of the preceding claims, **characterized in that** the solution for incubation in c) comprises at least one buffer substance and up to 500 mM salt, preferably up to 250 mM salt.

14. An assay kit for carrying out a method as claimed in any of claims 1 to 13, **characterized in that** it contains several Western blot strips or other supports on which antigens of a pathogen are immobilized, wherein at least two different antigens are immobilized in the same way and in the same quantity on each support, the different antigens on each support being spatially separate from one another.

15. An assay kit as claimed in claim 14, **characterized in that** it comprises a denaturing agent.

16. An assay kit as claimed in claim 15, **characterized in that** it comprises urea as denaturing agent.

17. The use of recombinantly produced antigens for determining the avidities of antibodies against antigens, wherein
a) at least two solid phases are provided, at least two different antigens being immobilized in the same way and in the same quantity on each solid phase, and the different antigens on each solid phase being spatially separate from one another,
b) the solid phases from a) are incubated with an antibody-containing solution,
c) one of the solid phases is incubated in a solution which negligibly reduces the binding of antibodies with low avidity to the antigens,
d) at least one other solid phase is incubated in a solution, where this solution comprises an agent which reduces the binding of antibodies with low avidity to the antigens, and
e) the antibodies bound to the solid phases are detected.

## Revendications

1. Procédé de détermination de l'avidité d'anticorps, qui comporte les étapes suivantes :
a) préparation d'au moins deux phases solides, au moins deux antigènes différents étant immobilisés sur chaque phase solide de la même manière et en même quantité, les différents antigènes étant séparés l'un de l'autre spatialement sur chaque phase solide,
b) incubation des phases solides provenant de a) avec une solution contenant des anticorps,
(c) incubation d'une des phases solides dans une solution, qui ne réduit sensiblement pas la fixation d'anticorps ayant une avidité faible sur les antigènes,
d) incubation d'au moins une autre phase solide dans une solution, cette solution présentant un agent qui réduit la fixation d'anticorps ayant une activité faible sur les antigènes, et
(e) détection des anticorps fixés sur des phases solides.

2. Procédé suivant la revendication 1, **caractérisé en ce que** l'agent mis en oeuvre dans l'étape d) contient au moins une substance dénaturant des protéines.

3. Procédé suivant la revendication 2, **caractérisé en ce que** l'agent contient de l'urée.

4. Procédé suivant la revendication 3, **caractérisé en ce qu'**on utilise de l'urée en une concentration de 4 à 9 M.

5. Procédé suivant l'une des revendications 1 à 4, **caractérisé en ce que** les phases solides sont des membranes.

6. Procédé suivant la revendication 5, **caractérisé en ce que** les membranes sont constituées d'une matière choisie parmi un groupe contenant de la nitrocellulose, du Nylon et du fluorure de polyvinylidène.

7. Procédé suivant l'une des revendications 5 et 6, **caractérisé en ce que** les membranes sont préparées par électrophorèse sur gel et ensuite transfert électrophorétique des antigènes sur les membranes.

8. Procédé suivant l'une des revendications 1 à 7, **caractérisé en ce que** la détection des anticorps dans l'étape e) est effectuée par incubation avec des anticorps secondaires couplés à une enzyme et réaction de mise en évidence enzymatique ultérieure.

9. Procédé suivant l'une des revendications précédentes, **caractérisé en ce que** l'avidité des anticorps est déterminée vis-à-vis du virus d'Epstein-Barr.

10. Procédé suivant l'une des revendications précédentes, **caractérisé en ce que** des protéines purifiées sont immobilisées sur les phases solides.

11. Procédé suivant l'une des revendications précédentes, **caractérisé en ce que** les antigènes immobilisés sur les phases solides sont préparés par recombinaison.

12. Procédé suivant l'une des revendications précédentes, **caractérisé en ce que**, après l'incubation, davantage d'anticorps ont été fixés sur la phase solide, qui a été incubée selon c), que sur la phase solide qui a été incubée selon d).

13. Procédé suivant l'une des revendications précédentes, **caractérisé en ce que** la solution destinée à l'incubation selon c) contient au moins une substance tampon et jusqu'à 500 mM de sel, de préférence jusqu'à 250 mM de sel.

14. Trousse d'essai pour la mise en oeuvre d'un procédé suivant l'une des revendications 1 à 13, **caractérisé en ce que** qu'elle contient plusieurs bandes de Western-Blot ou d'autres supports sur lesquels des antigènes d'un stimulateur sont immobilisés, au moins deux antigènes différents étant immobilisés sur chaque support de la même manière et en quantité identique, les différents antigènes étant mutuellement séparés spatialement sur chaque support.

15. Trousse d'essai suivant la revendication 14, **caractérisée en ce qu'**elle contient un agent dénaturant.

16. Trousse d'essai suivant la revendication 15, **caractérisée en ce qu'**elle contient de l'urée, comme agent dénaturant.

17. Utilisation d'antigènes préparés par combinaison pour la détermination des avidités d'anticorps vis-à-vis d'antigènes, dans laquelle
a) au moins deux phases solides sont préparées, au moins deux antigènes différents étant immobilisés sur chaque phase solide de la même manière et en quantité identique, les différents antigènes étant mutuellement séparés spatialement sur chaque phase solide,
b) les phases solides provenant de a) sont incubées avec une solution contenant des anticorps,
c) une des phases solides est incubée dans une solution, qui ne réduit sensiblement pas la fixation d'anticorps ayant une faible avidité sur les antigènes,
d) au moins une autre phase solide est incubée dans une solution, cette solution présentant un agent qui réduit la fixation d'anticorps ayant une faible avidité sur les antigènes, et
e) les anticorps fixés sur les phases solides sont détectés.
